# EUROPEAN PATENT APPLICATION

(11) **EP 3 871 678 A1**
(43) Date of publication of application: **01.09.2021**
(21) Application number: 20305186.7
(22) Date of filing: 26.02.2020
(51) Int. Cl.: A61K 31/7084, A61K 31/7072, A61K 31/7064, A61P 17/02, A61P 43/00

(54) **PRO-ANGIOGENIC COMPOUNDS**

(71) Applicant: UNIVERSITE DE BORDEAUX, 33000 Bordeaux (FR); Centre National de la Recherche Scientifique, 75016 Paris (FR); INSTITUT NATIONAL DE LA SANTE ET DE LA RECHERCHE MEDICALE (INSERM), 75013 Paris (FR)
(72) Inventor: BARTHELEMY, Philippe, 33700 Mérignac (FR); BOIZIAU, Claudine, 33450 Montussan (FR); BANSODE, Nitin, 413516 Killari (IN); CHASSANDE, Olivier, 33600 Pessac (FR); REMY, Murielle, 33640 Portets (FR); KOTAGUDDA RANGANATH, Sindhu, 33600 Pessac (FR); HAGEDORN, Martin, 33600 Pessac (FR)
(74) Representative: Santarelli

(57) **Abstract**

The invention relates to the use of low molecular weight amphiphilic compounds derived from nucleolipids for promoting angiogenesis.

## Description

The invention relates to the use of low molecular weight amphiphilic compounds derived from nucleolipids for promoting angiogenesis.

The design of hydrogels for regenerative medicine is often centered on cellular infiltration, degradation, and neovascularization. In this context, low molecular weight hydrogels (LMWHs), formed by self-assembly *via* non-covalent interactions, are gaining significant interest due to their soft nature, ease of use and injectability.

Amongst the biomaterials that are considered for clinical applications, injectable hydrogels have garnered significant interest due to their ease of use and tunable mechanical properties. As mostly formed of water, hydrogels are hydrophilic, can be highly swollen and facilitate the diffusion of oxygen and nutrients throughout their structure; some hydrogels are also good platforms for cell colonization. In addition, they can be injected and their local gelling allows for a tight adaptation to the local 3-D structure of the host environment. However, all these applications where a material is injected or implanted in the body are facing the same limitations: they must be biocompatible, and the initial structure as well as the degradation products must display as low cytotoxicity as possible. In addition, the inflammatory reaction that is elicited by the biomaterial must be controlled, firstly to prevent a too fast elimination/degradation of the biomaterial before successful tissue regeneration is reached, and secondly to avoid a strong and/or chronic local inflammatory environment. Indeed, any implantation or injection of a foreign body will trigger an inflammatory reaction called the "foreign body reaction" (FBR).

The use of VEGF clusters included in a hyaluronic acid-based gel containing heparin particles was studied as a tissue engineering approach for regenerating damaged brain, in Lina R. Nih et al., Springer Nature Switzerland AG 2018, Biophysical Regulation of Vascular Differentiation and Assembly, Chapter7, 177-187. In this study, it was shown that the effect on vascularization was related to the delivery of VEGF by the hyaluronic acid-based gel and that heparin particles helped generating functional axonal connections and reducing post-stroke inflammation.

Amphiphilic glucosylnucleosides (GNLs) are disclosed in WO2016/055493. WO2016/055493 also relates to hydrogels formed therefrom, as well as to their use as culture media, as biocompatible material for tissue engineering, cellular therapy or prevention of adhesion formation after abdomino-pelvic surgery, or else for control-release delivery of an active ingredient which is entrapped into the gel.

Hydrogels formed from GNLs or amphiphilic fluorinated glucosyl-nucleosides (GNFs), useful for removing particles from a liquid medium containing them, are disclosed in WO2013/110902.

However, the use of GNLs or hydrogels formed therefrom for controlling angiogenesis is not taught or suggested in the prior art.

It has now been found that GNLs or hydrogels formed therefrom, or degradation products deriving from said hydrogels could be used promoting angiogenesis tissue regeneration, in particular for tissue regeneration.

Advantageously, it was found that said amphiphilic glucosyl-nucleosides, in particular in the form of low molecular weight gels (LMWG) do not elicit foreign body reaction and show a good biointegration as evidenced by no sign of fibrous capsule after subcutaneous injection.

Without intending to be bound by any theory, it is contemplated that, by displaying a good biointegration without fibrosis, and by promoting efficient angiogenesis, which may be related, in particular to the release of a degradation by-product with angiogenic properties, amphiphilic glucosyl-nucleosides, in particular in the form of low molecular weight gels (LMWG), could be of major interest in various regenerative applications.

The invention thus relates to the use of amphiphilic glucosyl-nucleosides, in particular in the form of low molecular weight gels (LMWG) formed from therefrom, for controlling angiogenesis disorders.

The invention also relates to a method of preventing or treating angiogenesis disorders, by administration to a patient in need thereof.

According to a first aspect, the invention relates to a compound of formula (I)

R₁-X₁-A-X₂-R₂ (I)

in which
- X₁ is -NH-C(O)-NH- or -C(O)-NH-;
- X₂ is -NH-C(O)-NH- or -C(O)-NH- or does not exist;
- A is a C₄-C₃₀ hydrocarbon chain, linear or branched, saturated or unsaturated, which is unsubstituted or substituted by one or more C₁-C₁₂ linear or branched alkyl groups, or A represents a C₄-C₃₀ hydrocarbon chain, linear or branched, saturated or unsaturated, which is partially or completely halogenated;
- R₁ and R₂, identical or different, represent - (CH₂)ₙ-R₃-(CH₂)ₘ-R₄-(CH₂)ₚ-R₅-R₆ in which
   - n, m and p, identical or different, are 0 to 10;
   - R₃ represents a heteroaryl group comprising 1 to 4 oxygen or nitrogen atom(s);
   - R₄ represents a nucleosidyl group;
   - R₅ represents a heteroaryl group comprising 1 to 4 heteroatom(s);
   - R₆ represents a residue of a cyclic carbohydrate or a derivative of the said carbohydrate, and
- when X₂ does not exist, R₂ does not exist,
   provided that, when X₁ and X₂ are -NH-C(O)-NH-,n=2, m=p=1, R₃ is a triazole group, R₄ is thymidine and-R₆ is D-glucopyranosyl, ,
   for use in promoting angiogenesis.

Advantageously, the invention relates to a compound of formula (I) as defined above, for use in promoting angiogenesis for tissue regeneration, in particular for tissue regeneration assisted by a biomaterial.

"Tissue regeneration assisted by a biomaterial" means that a biomaterial can be implanted or injected in a tissue to promote, favor or improve its regeneration.

« Heteroaryl group containing 1 to 4 oxygen or nitrogen atoms » refers to a monocyclic or bicyclic, aromatic or partially unsaturated, carbocyclic group containing 5 to 12 atoms, interrupted by 1 to 4 oxygen or nitrogen atoms, which can be, for example, selected from furane, pyrrole, oxazole, oxadiazole, isoxazole, pyrazole, triazole, tetrazole, imidazole, pyridine, pyrimidine, pyridazine, pyrazine, benzofurane, indole, quinoleine, isoquinoleine, chromane, naphtyridine or benzodiazine groups, triazole being preferred.

« Hydrocarbon chain, which is partially or completely halogenated » refers to a saturated or unsaturated alkyl chain in which some or all hydrogen atoms are replaced by halogen atoms, such as fluorine, iodine, chlorine or bromine, fluorine being preferred.

« C₁-C₁₂ linear or branched alkyl group » refers, for example, to groups such as methyl, ethyl, propyl, isopropyl, butyl, isobutyl, sec-butyl, tert-butyl, pentyl, isopentyl, sec-pentyl, tert-pentyl, neo-pentyl, hexyl, isohexyl, sec-hexyl, tert-hexyl, heptyl, octyl, nonyl, decyl, undecyl or dodecyl, C₁-C₆ linear or branched alkyl groups being preferred.

« Nucleosidyl group » refers to a group consisting of a ribose or deoxyribose moiety which is linked to a purine or pyrimidine base, or to derivatives of said purine or pyrimidine base, or to a non-natural mono- or bicyclic heterocyclic base, all said bases being optionally substituted.

The purine base can be, for example, selected from the group consisting of adenine, guanine and hypoxanthine.

The pyrimidine base can be, for example, selected from the group consisting of thymine, uracile and cytosine, thymine being preferred.

By « non-natural mono-or bicycle heterocyclic base » is meant a universal base, such as, for example, 3-nitropyrrole, 4-nitroimidazole or 5-nitroindole.

By « optionally substituted » is meant that the purine or pyrimidine base, or the non-natural heterocyclic base can be substituted by at least one substituent chosen, for example, from a halogen, an amino group, a carboxy group, a carbonyl group, a carbonylamino group, a hydroxy, azido, cyano, alkyl, cycloalkyl, perfluoroalkyl, alkyloxy (for example, methoxy), oxycarbonyl, vinyl, ethynyl, propynyl, acyl group etc.

« Residue of a cyclic carbohydrate or a derivative of the said carbohydrate » refers to a residue of a 5- or 6- membered osidic cycle, which can be, for instance, selected from D-glucopyranose, D-galactopyranose, D-mannopyranose, D-fructopyranose or D-ribofuranose, or oligosaccharide-type glycans deriving therefrom, D-glucopyranose being preferred, or else to their, N-acyl derivatives, in particular their N-acetyl derivatives, such as, for instance, N-acetylglucosamine, N-acetylgalactosamine, N-acetylmannosamine or sialic acid, or a protected derivative thereof, such as an O-acyl derivative, in particular an O-acetyl derivative.

Preferred C₄-C₃₀ hydrocarbon chains are C₈-C₃₀, preferably C₈-C₂₄, more preferably C₁₂-C₂₀ hydrocarbon chains.

Preferably, A is a C₄-C₃₀, in particular C₈-C₂₄, more preferably C₁₂-C₂₀, hydrocarbon chain, linear or branched, saturated or unsaturated, which is unsubstituted or substituted by one or more C₁-C₁₂ linear or branched alkyl groups

Preferably, R₆ is an oligosaccharide-type glycan deriving from a cyclic carbohydrate.

The general and preferred aspects mentioned above apply to compounds of formula (I) and (II), and to their uses throughout the present description.

Preferred compound of formula (I) are those in which at least one of the following conditions is fulfilled:
- n=m=p=1
- R₃ and R₅, identical or different, represent a heteroaryl group containing 1 to 4 nitrogen atoms selected from the group consisting of pyrazole, triazole, tetrazole and imidazole, preferably triazole;
- R₄ represents a nucleosidyl group selected from adenosine, deoxyadenosine, guanosine, deoxyguanosine, thymidine, deoxythymidine, uridine, deoxyuridine, cytidine and deoxycytidine, preferably thymidine.
- R₆ represents a residue of a cyclic carbohydrate selected from D-glucopyranose, D-galactopyranose, D-mannopyranose, D-fructopyranose and D-ribofuranose, preferably D-glucopyranose.

A preferred compound is a compound of formula (I), in which:
- X₁ and X₂ are -NH-C(O)-NH-;
- A represents a C₁₂ saturated hydrocarbon chain;
- R₁ and R₂ are -(CH₂)ₙ-R₃-(CH₂)ₘ-R₄-(CH₂)ₚ-R₅-R₆;
- n = m = p = 1;
- R₃ is a triazole group;
- R₄ is thymidine;
- R₅ is a triazole group, and
- R₆ is D-glucopyranosyl.
This compound is called compound 17 in the examples.

Another preferred compound is a compound of formula (I) in which:
- X₁ and X₂ are -C(O)-NH-;
- A represents a C₁₄ saturated hydrocarbon chain;
- R₁ and R₂ are -(CH₂)ₙ-R₃-(CH₂)ₘ-R₄-(CH₂)ₚ-R₅-R₆;
- n = m = p = 1;
- R₃ is a triazole group;
- R₄ is thymidine;
- R₅ is a triazole group, and
- R₆ is D-glucopyranosyl.
This compound is called compound 20 in the examples.

Another preferred compound of formula (I) is defined as follows:
- X₁ is -C(O)-NH- and X₂ does not exist
- A represents a C₄-C₃₀ hydrocarbon chain, preferably C₈-C₂₄, linear or branched, saturated or unsaturated, which is partially or completely halogenated,
- R₁ represents - (CH₂)ₙ-R₃-(CH₂)ₘ-R₄-(CH₂)ₚ-R₅-R₆, as defined above.

A preferred compound of formula (I) in which :
- A represents a saturated linear C₁₀ hydrocarbon chain, which is completely halogenated and the halogen atom is fluorine,
- X₁ is -C(O)-NH- and X₂ does not exist- R₁ represents - (CH₂)ₙ-R₃-(CH₂)ₘ-R₄-(CH₂)ₚ-R₅-R₆ in which n=m=p=1, R₃ is a triazole group, R₄ is thymidine, R₅ is a triazole group and R₆ is D-glucopyranosyl,
is called GNF in the examples.

In particular, in formula (I), R₃ is covalently linked to R₄ to the carbon atom at position 5' of the ribose or deoxyribose ring of the nucleosidyl group.

In particular, in formula (I), R₅ is covalently linked to R₄ via (CH₂)p to the azote atom at position 3 of the purine or pyrimidine base moiety, for example the thymine moiety, of the nucleosidyl group.

Advantageously, the following compounds of formula (I) are used according to the invention:
- 1,12-bis-dodecanyl-5'-[(4-methylurea)-1*H*-1,2,3-triazol-1-yl]-N-3-[1-((β-D-glucopyranoside)-1H-1,2,3-triazole-4-yl)methyl]-5'-deoxy thymidine,
- 1,14-bis-tetradecanyl-5'-[(4-methylamide)-1*H*-1,2,3-triazol-1-yl]-N-3-[1-((β-D-glucopyranoside)-1H-1,2,3-triazole-4-yl)methyl]-5'-deoxy thymidine,
these compounds being respectively disclosed as compounds 17 of example 7 and compound 20 of example 9 in WO2016/055493 (referred to as "compound 17" and "compound 20" in the examples), and
5'-(4-((2H,2H,3H,3H-perfluoroundecanamide)methyl-1H-1,2,3-triazol-1-yl)-N3-(1-((β-D-glucopyranoside)-1H-1,2,3-triazole-4-yl)methyl)thymidine,
this compound being disclosed in WO2013/110902 (referred to as "GNF" in the examples).

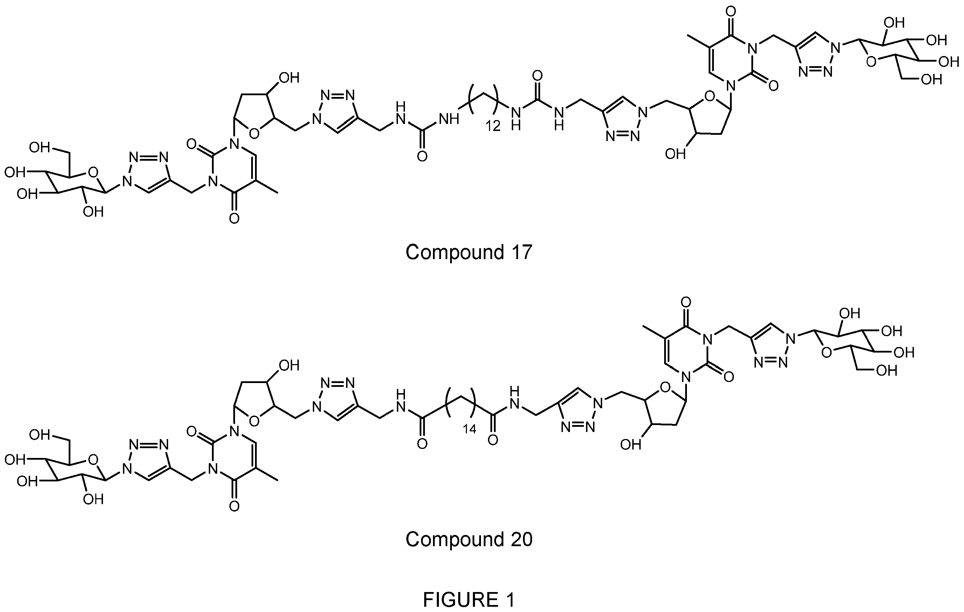

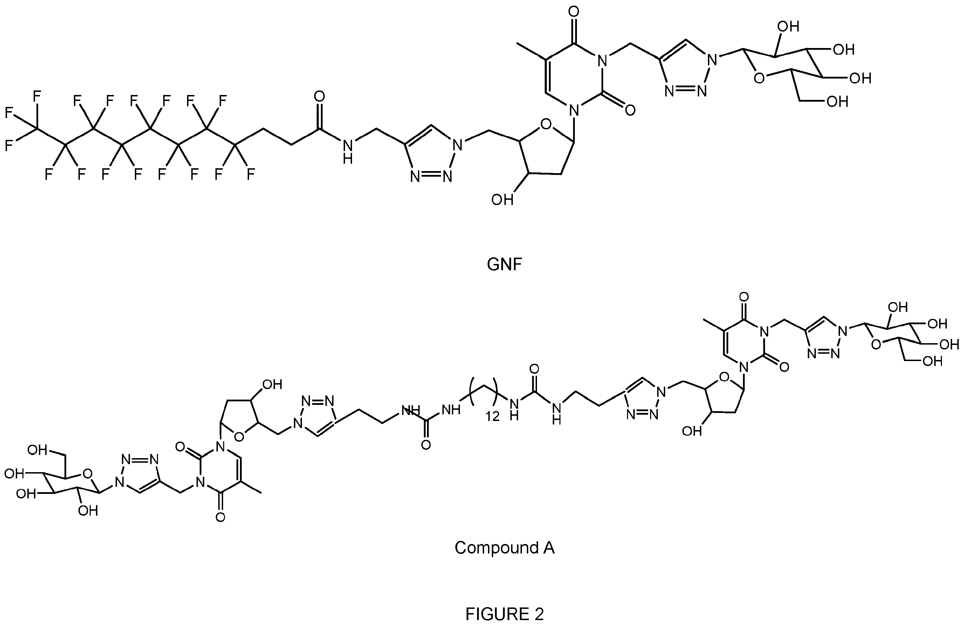

Compounds 17 and 20, as well as GNF, are shown on Figures 1 and 2.

According to a preferred aspect, the compounds of formula (I) as defined above can be used in the form of a hydrogel.

The invention also relates to a compound of formula (II)

R₄-(CH₂)ₚ-R₅-R₆ (II)

in which:
- p is 0 to 10;
- R₄ represents a nucleosidyl group;
- R₅ represents a heteroaryl group comprising 1 to 4 heteroatom(s);
- R₆ represents a residue of a cyclic carbohydrate or a derivative of the said carbohydrate,
for use in promoting angiogenesis.

The general and preferred definitions of R₄, R₅ and R₆ are as defined for formula (I).

Preferably, in formula (II):
- p = 1;
- R₄ is thymidine, cytidine or adenosine, thymidine being preferred;
- R₅ is a triazole group, and
- R₆ is D-glucopyranosyl,
for use in promoting angiogenesis.

A preferred compound of formula (II) is the compound referred to as GN-1 in the examples, where:
- p = 1;
- R₄ is thymidine;
- R₅ is a triazole group, and
- R₆ is D-glucopyranosyl.

This compound is represented by formula (IIa) below:

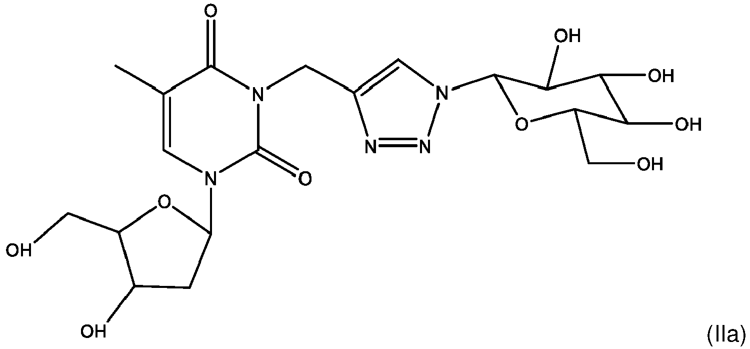

Advantageously, the invention relates to a compound of formula (II) as defined above, in particular a compound of formula (IIa), for use in promoting angiogenesis for tissue regeneration, in particular for tissue regeneration assisted by a biomaterial.

The preparation of compounds of formula (II), in particular of formula (IIa) as described above, in the form of a protected tetra-acetylated glucosyl derivative, was reported in WO2016/055493 and in M.A. Ramin et al., 2017, Biomaterials doi .1016/j.biomaterials.2017.08.034.

Deacetylation can be performed by usual techniques, such as, for example by using sodium methoxide in an anhydrous methanol solution, and addition of an ion-exchange resin, as disclosed in WO2016/055493.
Figure 1 shows compound 17 and compound 20.
Figure 2 shows GNF and compound A, which is similar to compound 17, except that it has an additional carbon bond between the urea group and the triazole ring. This compound is used as a control.Figure 3 shows the analysis of in vitro degradation of hydrogels formed from compound 17, compound 20, GNF and compound A.
Figures 4A, 4B, 5A and 5B show the MALDI-TOF Mass spectra of molecules released during the 21-day incubation in PBS at 37°C of compound 17, compound 20 ,GNF and compound A.
Figure 6 shows the locations were possible fragmentation occurs in compound 17, compound 20 and GNF by bond cleavage, as shown by the thick arrows.
Figure 11 shows the quantitative analysis of blood vessels counted in the vicinity of the hydrogels formed from compound 17, compound 20 and GNF as compared with the control compound A.
Figures 12A and 12B show the quantification of the presence or absence of sprouting and tortuous vessels in an in vivo chick chorioallantoic membrane (CAM) assay with compound GN-1.

The invention is illustrated by the following examples.

Data are presented as mean ± SD. Differences between groups were evaluated using Kruskal-Wallis (for at least three compared groups) and Mann-Whitney tests (for two compared groups) for non-parametric measures. To analyze the repeated measures of hydrogel degradation follow-up, Friedman test was used; the comparison of paired values was then done with the use of the Wilcoxon matched-pair test. The Fisher exact test was used for analysis of angiogenesis on CAM. Two-tailed *p* values less than 0.05 were considered as significant.

### Example 1: Study of the in vitro degradation of the hydrogels formed from compounds of formula (I)

### a) Hydrogel preparation

Hydrogels were prepared with compound 17, compound 20, GNF at a concentration of 1.5% (w/v) by dissolving the powder in sterile phosphate buffered saline solution (PBS 1X, pH 7.4). The suspensions were heated at 65 °C for GNF and 80°C for the compounds 17 and 20. The GNF solution was incubated at 37 °C for 30 min to form a gel, whereas the other molecules formed the gel within 5 min.

Hydrogels were also prepared from compound A, which was used as a control.

Compound A, shown on Figure 2, is similar to compound 17, except that it has an additional carbon bond between the urea group and the triazole ring. Compound A can form a hydrogel but does not show the proangiogenic properties of Compounds 17, 20 and GNF, as will be explained below.

### b) In vitro degradation of the hydrogels

Hydrogel disks (100 µl) were prepared and were incubated in 7 ml of PBS 1X at 37°C for 21 days. All the hydrogel samples were weighed before incubating with the phosphate buffer (initial weight). The samples were removed at different time points (1, 2, 3, 5, 7, 15, and 21 days), weighed immediately after removing the excess of PBS by gently touching the surface of a tissue (Kimwipes™) and put back in the same solution of PBS.

The degradation products generated during the 21-day incubation of the samples was determined by analyzing the PBS solution in which they were incubated. At day 21 the PBS was collected and analyzed for the degradation products of individual hydrogels by using mass spectroscopy. Studies, performed in triplicates, allowed for the calculation of degradation kinetics.

### c) Results

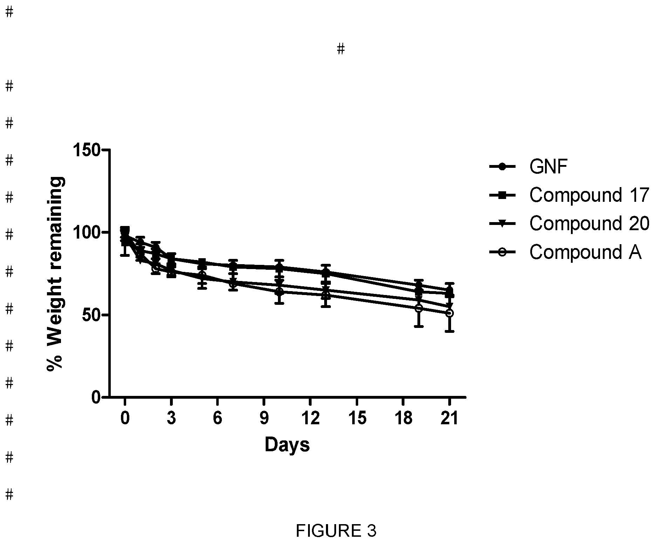

The degradation was monitored by measuring the weight loss of the gels after different periods of incubation. After 3 weeks at 37°C, the remaining weights were 65 ± 4 %, 63 ± 1 %, and 55 ± 2 %, for GNF, compound 17 and compound 20, respectively, as shown on Figure 3..

The control compound A has a similar degradation kinetics, but no fragmentation is observed with this control compound.

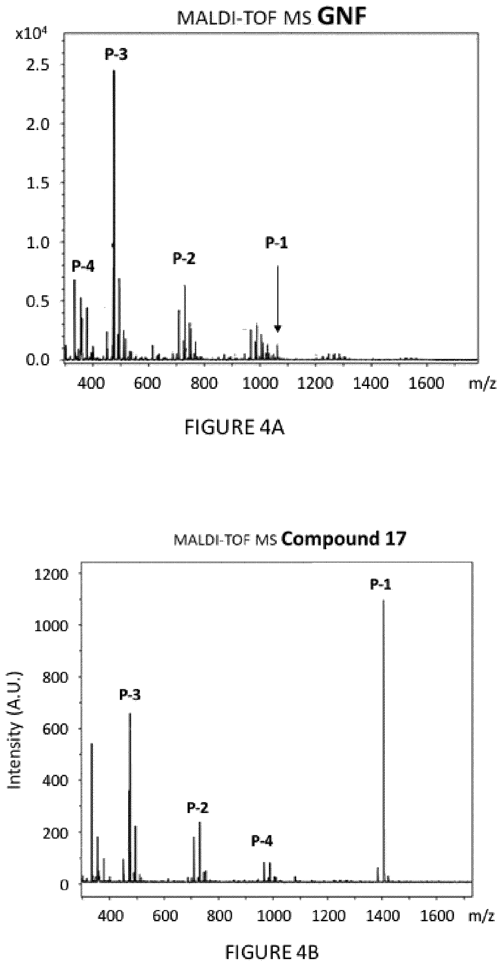

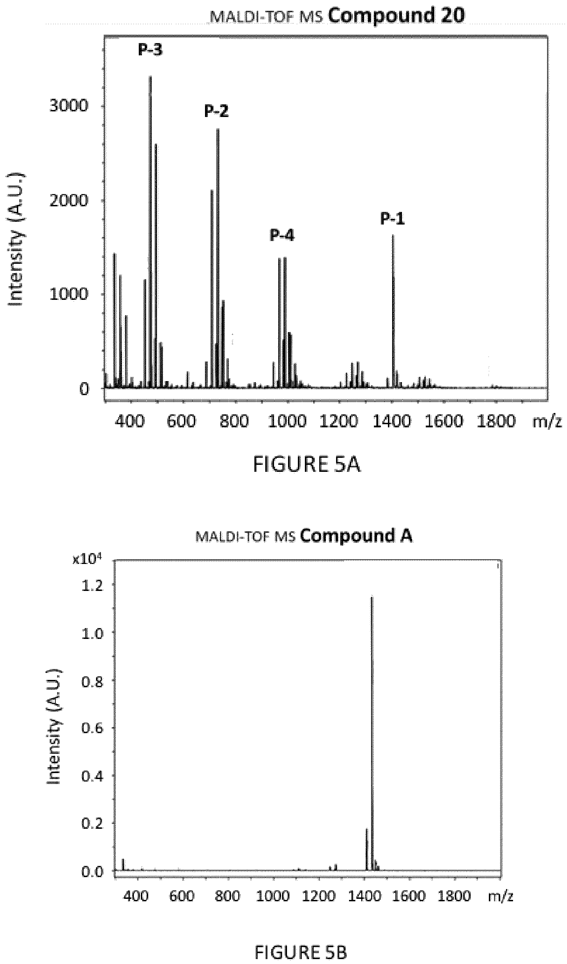

In order to assess the fragmentation of the molecules during incubation, the by-products of degradation present in the buffer were analysed by MALDI-TOF (Matrix Assisted Laser Desorption Ionization - Time of Flight) mass spectrometry at the end of the experiment (21 days) (Figures 4 and 5).

The analyses were carried out on the MALDI-TOF-TOF Ultraflex (Bruker) in positive reflector mode (detection of positively charged species in the form of [M + H] + and [M + Na] + ions).The external calibration of the spectrometer was carried out with a mixture of components in the mass range m / z 700-2500. The matrix used is a solution of 2,4,6 trihydroxyacetophenone at 20 mg / ml in EtOH / NH₄ citrate 25 mM. The samples were diluted by a factor of 50 in water before MS analysis.

### d) Analysis of the products released by fragmentation of the hydrogels

The analysis of the products released in PBS by the hydrogels formed from GNF (Figure 4A), compound 17 (Figure 4B), compound 20 (Figure 5A) and compound A (Figure 5B) after the 21-day incubation showed the molecular peaks **(P-1)** corresponding to intact molecules: their mass was in close agreement with the calculated mass. Other peaks **P-2, P-3,** and **P-4** were observed in the mass spectra of the products released from hydrogels GNF, compound 17 and compound 20 which were due to fragmentations of the hydrogel molecules as a result of degradation.

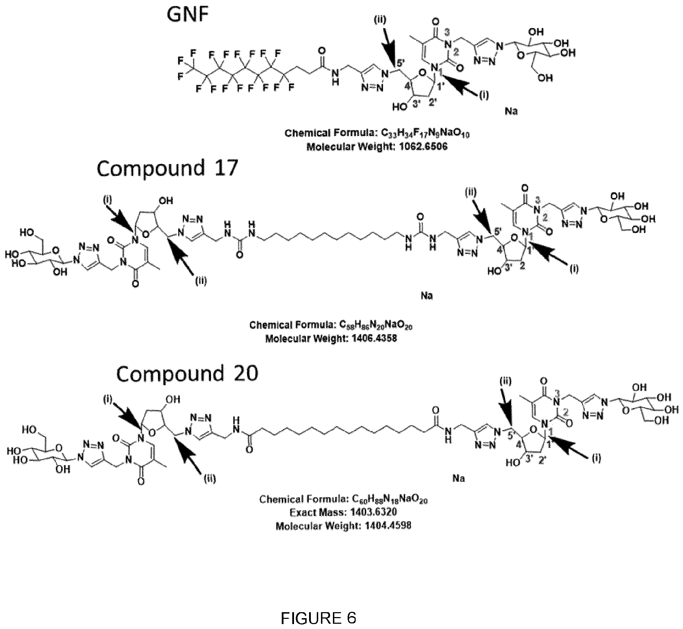

It was hypothesized that the first possible fragmentation of hydrogels is due to the cleavage of the bond between the C1'-carbon of pentose ring and the N1'- nitrogen of the nucleobase (Figure 6, thick arrow (i), **Peak 2** in mass spectrum); the second possible fragmentation is due to the bond cleavage between bridged 5'-CH₂ and pentose ring (Figure 6, thick arrow (ii), **Peak 3** in mass spectrum). Calculated mass and observed mass for the fragments due to possible cleavages are summarized in Table 1 below. In comparison, only the intact molecule of compound A was detected, showing that this control compound disassembles without degradation (Figure 5B).

In contrast to hydrogels formed from compounds 17, 20 and GNF that release degradation products (shown by peaks P-2, P-3, and P-4), there is no production of degradation products with hydrogels formed from control compound A (only peak P-1 is detected) (Figure 5B).

**Table 1**

| **Compound** | **Fragmentation** | **Cleavage** | **Calculated mass** | **Observed mass** |
|---|---|---|---|---|
| **GNF** | First fragmentation (I) | C1'-N1 | Fragment-1: 734.41 | 731.12[M+Na+K]⁺(Peak 2) |
| | | | [M+Na+K]⁺ | 368.00[M]⁺(Peak 4) |
| | | | Fragment-2: 369.33[M]⁺ | |
| | Second fragmentation (II) | CH₂-Methylene CH₂ | Fragment-3: 492.42[M+Na]⁺ | 495.04[M+Na]⁺(Peak 3) |
| **Compound 17** | First fragmentation (I) | C1'-N1 Methylene | Fragment-2: 733.68 | 731.17[M+2Na+K]⁺ (Peak 2) |
| | | | [M+2Na+K]⁺ | |
| | Second fragmentation (II) | C4-Methylene | Fragment-4 : 476.33[M]⁺ | 475.47[M]⁺ (Peak 3) |
| | | CH₂ | | |
| | | | Fragment-1: 969.13[M+K]⁺ | 967.21 [M+K]⁺(Peak 4) |
| **Compound 20** | First fragmentation (I) | C1'-N1 Methylene | Fragment-1 : 736.40 [M+Na+K]⁺ | 731.13[M+Na+K]⁺(Peak 2) |
| | Second fragmentation (II) | C4-Methylene | Fragment-3 : 474.65[M] | 473.06[M]⁺(Peak 3) |
| | | CH₂ | Fragment-2: 990.15 [M+Na+K]⁺ | 989.16[M+Na+K]⁺(Peak 4) |
| **Compound A** | No fragmentation observed | | | |

### Example 2: Study of the in vitro non-specific protein adsorption on hydrogels formed from compounds of formula (I)

### a Protocol

The non-specific protein adsorption was investigated by using bovine serum albumin (BSA, GE-healthcare ref K45-001) as a model protein. The adsorption of BSA was evaluated by standard bicinchoninic acid protein assay (BCA). The formed hydrogels were cut into disks (100 µl) followed by incubation in PBS overnight at 37°C to reach a swelling equilibrium. On the following day, the PBS was replaced by 400 µL PBS containing BSA (2 mg/ml, freshly prepared), followed by incubation at 37 °C for 2 h.

Further, the samples were rinsed thoroughly several times with PBS to remove unbound BSA proteins. Then, 400 µl of sodium dodecyl sulfate solution 1 % (w/v) was added into each well and soaked for 30 min at room temperature to strip the proteins adsorbed on the surface of the hydrogels for detection. The striped protein was analyzed using the BCA protein kit (Pierce™ BCA Protein Assay Kit) as recommended by the manufacturer, to determine the amount of BSA adsorbed on the hydrogels. The amount of protein was calculated according to the calibration curve of BSA. The negative control was done in a similar way excluding the incubation.

### b) Results

Statistical analysis with Kruskal-Wallis followed by Mann-Whitney tests (2-tailed) was performed to compare the adsorption of BSA to the three hydrogels (mean ± s.d, n = 3, **p* ≤ 0.05).

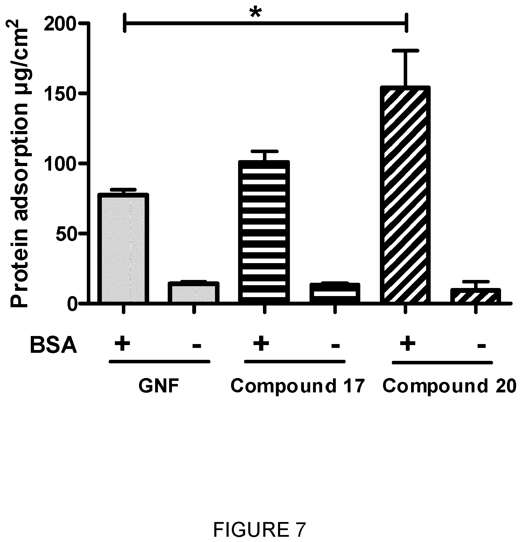

As shown in Figure 7, compound 20 showed significantly higher protein adsorption (118±21 ng/cm²) compared to GNF (60±3µg/cm², *p* = 0.05, 2-tailed). compound 17 had intermediate BSA adsorption (78±6µg/cm²), whereas the hydrogels incubated without BSA gave no signal in BCA assay.

The low non-specific protein adsorption of GNF and compound 17 are predictive for minimizing the foreign body reaction upon in vivo implantation.

### Example 3: In vitro cytoxicity assays

### a) Protocol

The cytotoxicity evaluation of hydrogels was assessed according to NF-EN30993-5 and ISO10993-5 with some modifications.

MTT and Neutral Red assays were carried out to assess the viability and metabolic activity of cells.

The L929 cell line and adipose tissue derived mesenchymal stromal cells (MSCs), cultured in alpha-MEM (Gibco, Ref A10490-01, France) without ascorbic acid, and containing 10% (v/v) fetal calf serum (FCS, Lonza, France), were used for testing the cytotoxicity of the extracts. The cells were seeded at a density of 10,000 cells/cm² in 24-well microtiter plates (Nunc, Denmark) and the culture was maintained at 37°C for 72 h after cell plating.

At subconfluency, hydrogel pieces put into Falcon inserts (Ref:353096, 24 well format) were placed on the top of cell culture wells so that the gel pieces were immersed in the culture medium. The ratio of the sample surface area to the volume of the vehicle was 5 cm²/ml. In addition, wells containing alpha-MEM + 10% FCS alone or containing 0.1% Triton X100 (known to be cytotoxic), were processed under the same conditions to provide negative and positive controls of cytotoxicity, respectively. Culture plates were then kept in the incubator at 37°C for 24 hours, giving the "24(1)" cell culture; then, the inserts were transferred on other subconfluent L929 and MSC cultures for a second and then a third incubation of 24h, giving "24(2)" and "24(3)" cell cultures.

At the end of each 24h incubation, cell viability was assessed by the Neutral Red assay and cell metabolic activity by using an MTT assay. The MTT (Sigma-Aldrich, ref M2128) of 5mg/ml stock solution was diluted to 1/5 with alpha-MEM (without serum) without phenol red and 125 µl of this solution was added to each well. After 3 hrs of incubation at 37°C in 5% CO2, the supernatant was removed and formed formazan crystals were dissolved by adding 100 µl of dimethyl sulfoxide (DMSO, Ref D5879-1L, Sigma-Aldrich, France).

For the viability assay, the neutral red (Ref N4638, Sigma-Aldrich, France) was resuspended at 1.25% (w/v) in alpha-MEM without serum, and 100µl of the prepared solution was added to each well. After 3 hrs of incubation at 37°C, the supernatant was replaced by adding 100µl of mixture (50:50) containing 50% ethanol/ 1% acetic acid in water to lyse the cells. In both cases, the intensity of colour was quantified by measuring the absorbance at 540 nm using a spectrophotometer (Perkin Elmer ®, 2030 Multilabel Reader VictorTMX3). The absorbance is directly proportional to the viability and metabolic activity of the cell population and inversely proportional to the toxicity of the products released by the materials. The mean values of absorbance measurements obtained from colourimetric tests and their corresponding standard deviation (± SD) were calculated. The results are expressed as a percentage of the control (alpha-MEM + 10% FCS).

### b) Results

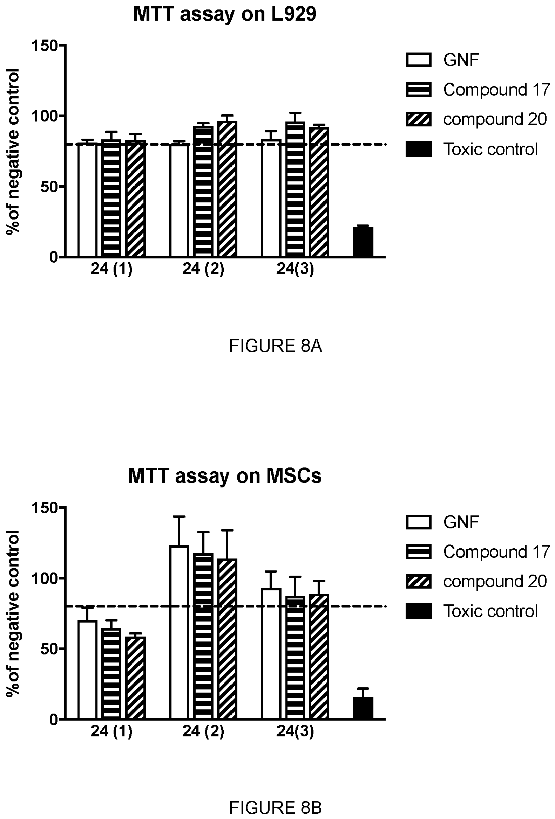

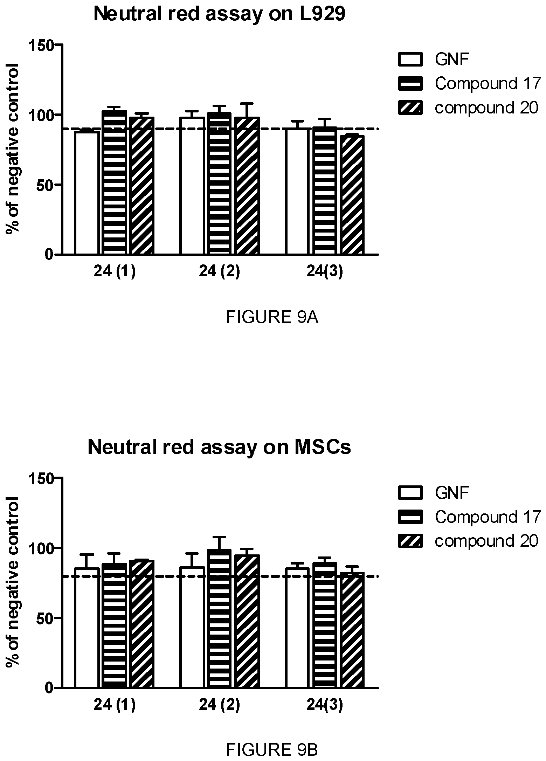

The results are shown on Figures 8 and 9.

Cell viability and metabolism were normalized against control alpha-MEM medium, whereas 0.1% Triton-X100 served as a positive control for cytotoxicity. The viability of L929 cells and MSCs, and the metabolism of L929, cultured with molecules released in contact of hydrogels exhibited no cytotoxicity; indeed, no significant difference was observed in comparison to the control of alpha-MEM medium, *i.e.,* for all the test groups, they were above 80 % (shown by the dotted line) representing the cut value of cytotoxicity (Figures 8A, 8B, 9A and 9B). In contrast, a low decrease of MSC metabolism was observed with the hydrogels during the first 24 hours: metabolism was between 60 and 70% as shown by the MTT assays (Figure 8B).

### Example 4: In vivo toxicity assays with hydrogels formed from compounds of formula (I)

### a) Protocol

### - Subcutaneous injection in mice

10-week-old OF-1 female mice (Charles Rivers, France) were used for this study. Before the bilateral subcutaneous injection of hydrogels, mice were anaesthetized using 4.5% isoflurane gas and maintained throughout surgery at 2.5% isoflurane with a face mask. The animals were depilated before surgery. The three different hydrogels were prepared as mentioned above and collected in 1 ml syringes, when the solution was in viscous form. The hydrogels were injected subcutaneously by 21-gauge needle on the dorsal side of the animals.

### - In vivo toxicity assays

The in vivo toxicity of the hydrogels formed from compounds 17, 20 and GNF was investigated by serum biochemical evaluation such as alanine aminotransferase (ALT Activity Assay Kit, Sigma-Aldrich, ref MAK052), aspartate aminotransaminase (AST Activity Assay Kit, Sigma-Aldrich, ref MAK055), and lactate dehydrogenase (LDH Assay Kit, Abcam, ref ab102526). At day 0 before surgery and three-time points after implantation (3, 7, and 21 days), blood was collected from retro-orbital sinus. The blood samples were allowed to clot then followed by centrifugation to collect the serum. The serum was used for analysis as recommended by the manufacturers.

### b) Results

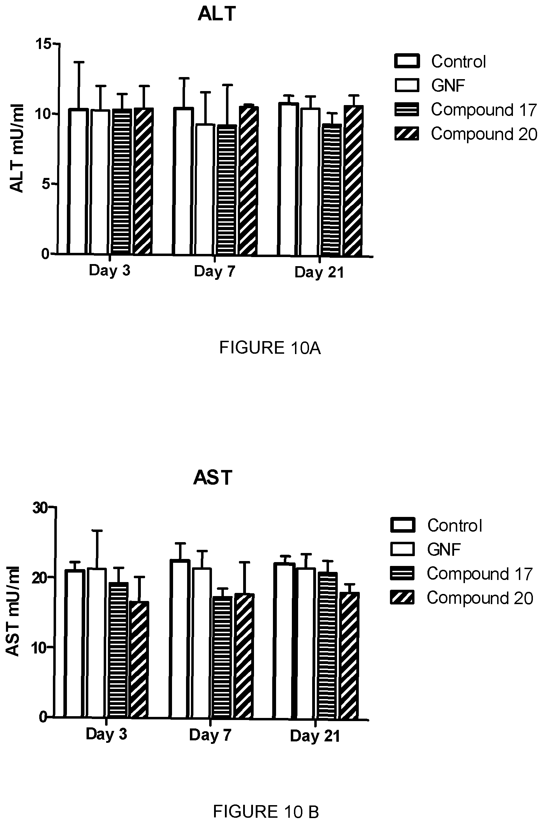

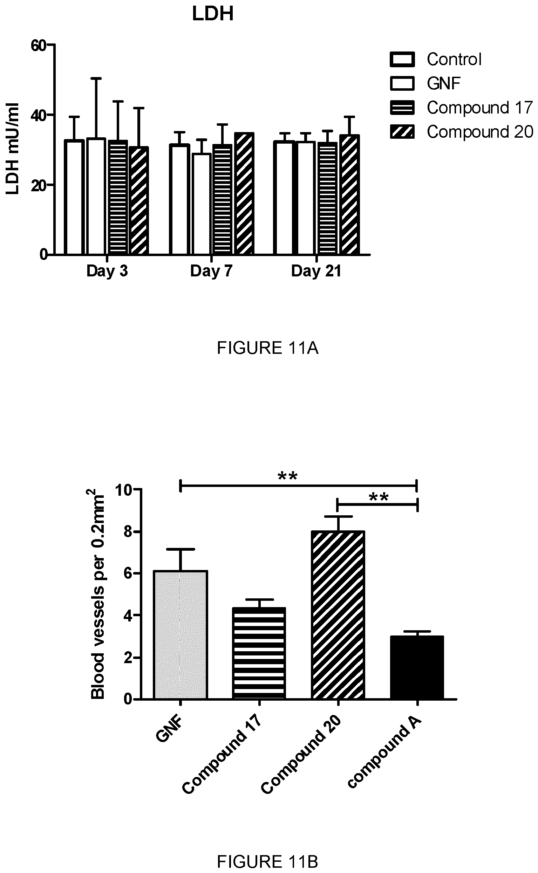

The results are shown on Figures 10A, 10B and 11A.

The hydrogels injected subcutaneously in mice and their effect on vital organs was investigated. The systemic toxicity was assessed by measuring biochemical serum parameters at three time points, day 3, day 7 and day 21 following the injection. The time points were selected based on the *in vitro* degradation behaviour of the hydrogels showing that, by day 3, 70-80% of the hydrogels remained whereas after 21 days, between 35% and 50% of the hydrogels were degraded. The results show that the injected hydrogels did not produce any significant variation neither in hepatic function markers such as serum alanine transaminase (ALT) as shown on Figure 10A or serum aspartate transaminase (AST) as shown on Figure 10B, nor in the cardiac toxicity marker lactate dehydrogenase (LDH) as shown on Figure 11A. Compared with the control serum levels in non-injected mice, all the indicative markers remained unchanged from the normal clinical range. This confirms that the tested hydrogels (formed from GNF, compound 17, compound 20) did not show any toxicity to the vital organs.

### Example 5 : Study of in vivo proangiogenic properties of the hydrogels formed from compounds of formula (I) by histology, staining and immunochemistry

### a) Protocol

Subcutaneous injection was carried out, as indicated in example 3, in order to characterize the behavior of the hydrogels after subcutaneous injection and *in situ* gelation. Animals were sacrificed by cervical dislocation one or three weeks after injection of the hydrogel. The biomaterials (i.e. hydrogel) together with the skin surrounding the injection point were harvested after 7, or 21 days and embedded in paraffin. Histological sections of skin portions associated to the hydrogel were then stained to assess the degradation pattern, cell infiltration (data not shown) and angiogenesis.

The blood vessels in and around the hydrogels 21 days after implantation were identified by immunolabelling with rabbit anti-CD31 antibody (Abcam, ref ab28364 dilution:1/100) followed by incubation with HRP-conjugated anti-rabbit secondary antibody (Vector MP-7401, dilution:1/2)

### c) Results

During the regeneration process, the development of neovessels in the remodeling tissue is of major importance. Therefore, immunostaining of CD31, an endothelial cell marker, was performed on day 21 to assess angiogenesis.

CD31 positive cells with luminal structure were observed in close proximity of the hydrogels, and in the tissue that had invaded the degrading hydrogels. CD31 positive cells were not found inside the hydrogels, showing that the non-degraded hydrogels were bad substrates for blood vessel ingress. Figure 11B depicts the quantification of blood vessels counted manually per 0.2mm² at four different random regions in the tissue surrounding each hydrogel. With the hydrogel formed from the control compound A (that disassembles without molecule degradation,Figure 5B), there was a 2.7-fold and 2.1-fold reduction in the numbers of blood vessels (bv) in the vicinity of this hydrogel (2.95 ± 0.27 bv/0.2 mm2) as compared to in the vicinity of hydrogels formed from compound 20 (7.95 ± 0.74 bv/0. 2 mm2, p=0.009), and from compound GNF (6.10 ± 1.07 bv/0.2 mm2, p=0.009), respectively. The hydrogel formed from compound 17 induced intermediate angiogenesis (4.30±0.41 bv/0.2mm², *p*>0.05).

### Example 6: preparation of N-3-[1-((β-D-Glucopyranoside)-1H-1, 2, 3-triazol-4-yl) methyl] thymidine (compound GN-1)

This compound was hypothesized as mimicking a degradation product of the tested hydrogels formed from compound 17, compound 20 or GNF, based on the degradation analysis of example 1.

This compound was prepared starting from the N-3-[1-((β-D-Glucopyranosidetetraacetate)-1H-1, 2, 3-triazol-4-yl) methyl] thymidine described as compound 7 in M. A. Ramin et al., Biomaterials, 2017, 145, 72-80, by the following procedure :
To the stirred solution of N-3-[1-((**β**-D-Glucopyranosidetetraacetate)-1H-1, 2, 3-triazol-4-yl) methyl] thymidine (0.7 g, 1.07 mmol) in anhydrous methanol (10 mL) was treated with freshly prepared 1N sodium methoxide solution at room temperature. After 1h stirring, Amberlite IRC-50 ion exchange resin was added to the reaction mixture and stirred for 30 min and filtered. After filtration, the solvent was removed under reduced pressure. The solid residue was applied to a column of silica gel, and the product eluted with DCM:MeOH (100 to 8:2) to afford the title compound as a white amorphous solid 0.5 g (96%).
**¹H NMR (CDCl₃, 300 MHz) δ** 8.12 (s, 1H, H triazole), 7.89 (s, 1H, H-6 thymine), 6.36-6.31 (t, *J*= 9 Hz, 1H, H-1'), 5.60-5.57 (d, *J*= 9 Hz, 1H, H-1), 5.29-5.19 (m, 2H, NCH₂triazole), 4.43-4.39 (m, 1H, H-4'), 3.94-3.68 (m, 6H, H3', H2, H3, H4, H5, H6'), 3.46-3.60 (m, 3H, H6', H6), 2.34-2.15 (m, 2H, H-2'), 1.93 (s, 3H, CH₃ thymine), ¹³C **NMR** (**CDCl₃**, 75 **MHz) δ** 163.54, 150.74 (C=O thymine),143.26 (Cq triazole), 135.36 (C-6 thymine), 122.85 (CH triazole), 109.36 (C-5 thymine), 88.19, 87.45(C-4', C-1'), 85.76 (C-1), 79.69 (C-5), 76.98, 72.53, 70.67 (C-3', C-3, C-2), 69.44 (C-4), 61.33, 60.96 (C-6, C-5'), 39.90 (C-2'), 35.66 (triazole CH₂N thymine), 11.79 (CH₃ thymine);
HRMS (m/z): Calculated [M+Na] + (C₁₉H₂₇N₅O₁₀Na) 508.1656, found 508.1650.

### Example 7 : Study of in vivo proangiogenic properties of compound GN-1 of example 5 by the Chick chorioallantoic membrane (CAM) assay

### a) Protocol

The angiogenesis assay was performed on the CAM of chick embryos (Gallus gallus) (E.A.R.L. Morizeau, Dangers, France). Embryos were incubated at 37°C and 70% humidity to initiate the fertilization. On day 3 of development, 3-5 ml of egg white was withdrawn by punctuating the air chamber with the syringe, and a window was made by cutting the eggshell and sealed with Durapore® tape. On day 13, prepared plastic rings (made from Nunc Thermanox® coverslips, diameter 13 mm and thickness 0.2mm) were placed on the intact CAM. 20 µl of the molecule (concentration 500 µg/ml in DMEM medium) or an equivalent volume of DMEM alone as control were deposited in the center of the plastic rings. The treatment was repeated the following day followed by photo documentation (day 14). On day 17, the eggs were fixed by addition of 2 ml of 4% paraformaldehyde (Antigenfix) for 30 min at room temperature. Imaging was performed with a stereomicroscope (Nikon SMZ800) using a digital camera (Nikon Coolpix 950). The images were scored for morphological changes of blood capillaries such as sprouting and tortuosity (0=normal, 1=overrepresented) by five observers using a blinded screening test (data not shown).

### b) Results

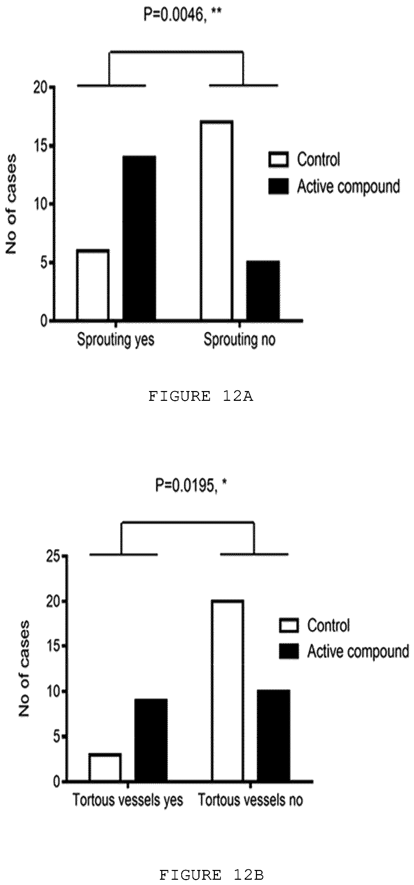

The angiogenic response was evaluated on day 17 based on two morphological parameters: sprouting, and presence of tortuous vessels (data not shown). In some CAMs, the presence of sprouting at the capillaries was observed, and of tortuous capillaries, whereas some other CAMs exhibited normal vasculature (data not shown). The quantification of eggs with or without sprouting, and/or tortuous vessels showed that more CAMs that were treated with GN-1 exhibited sprouting and tortuous vessels compared to the control CAMs (Figures 12A and 12B, "sprouting yes" and "tortuous vessels yes", respectively). In line with these results, CAMs without sprouting or without tortuous vessels were more frequent in the control group (Figures 12A and 12B, "sprouting no" and "tortuous vessels no", respectively). The differences between treated and non-treated CAMs were statistically significant: *p* = 0.0046 and *p* = 0.0195, for the sprouting and tortuous vessel effects, respectively (Fisher's exact test).

The tortuosity of neovessels that was evidenced with the *in vivo* CAM model treated with GN-1 shows that angiogenesis is promoted.

## Claims

1. A compound of formula (I)
R1-X1-A-X2-R2 (I)
in which
- X₁ is -NH-C(O)-NH- or -C(O)-NH-;
- X₂ is -NH-C(O)-NH- or -C(O)-NH- or does not exist;
- A is a C₄-C₃₀ hydrocarbon chain, linear or branched, saturated or unsaturated, which is unsubstituted or substituted by one or more C₁-C₁₂ linear or branched alkyl groups, or A represents a C₄-C₃₀ hydrocarbon chain, linear or branched, saturated or unsaturated, which is partially or completely halogenated;
- R₁ and R₂, identical or different, represent - (CH₂)ₙ-R₃-(CH₂)ₘ-R₄-(CH₂)ₚ-R₅-R₆
in which
• n, m and p, identical or different, are 0 to 10;
• R₃ represents a heteroaryl group comprising 1 to 4 oxygen or nitrogen atom(s);
• R₄ represents a nucleosidyl group;
• R₅ represents a heteroaryl group comprising 1 to 4 heteroatom(s);
• R₆ represents a residue of a cyclic carbohydrate or a derivative of the said carbohydrate, and
- when X₂ does not exist, R₂ does not exist,
provided that, when X₁ and X₂ are -NH-C(O)-NH-, n= 2, m=p=1, R₃ is a triazole group, R₄ is thymidine and-R₆ is D-glucopyranosyl, A is not a C₁₂ unsaturated hydrocarbon chain,
for use in promoting angiogenesis.

2. A compound of formula (I) for use according to claim 1, for tissue regeneration.

3. A compound of formula (I) for use according to claim 1 or 2, for tissue regeneration assisted by a biomaterial.

4. A compound of formula (I) for use according to any one of claims 1 to 3, in which R₆ is an oligosaccharide-type glycan deriving from a cyclic carbohydrate.

5. A compound of formula (I) for use according to any one of claims 1 to 4, in which at least one of the following conditions is fulfilled:
- n=m=p=1
- R₃ and R₅, identical or different, represent a heteroaryl group containing 1 to 4 nitrogen atoms selected from the group consisting of pyrazole, triazole, tetrazole and imidazole;
- R₄ represents a nucleosidyl group selected from adenosine, deoxyadenosine, guanosine, deoxyguanosine, thymidine, deoxythymidine, uridine, deoxyuridine, cytidine and deoxycytidine.
- R₆ represents a residue of a cyclic carbohydrate selected from D-glucopyranose, D-galactopyranose, D-mannopyranose, D-fructopyranose and D-ribofuranose.

6. A compound of formula (I) for use according to any one of claims 1 to 5, in which:
- X₁ and X₂ are -NH-C(O)-NH-;
- A represents a C₁₄ saturated hydrocarbon chain;
- R₁ and R₂ are -(CH₂)ₙ-R₃-(CH₂)ₘ-R₄-(CH₂)ₚ-R₅-R₆;
- n = m = p = 1;
- R₃ is a triazole group;
- R₄ is thymidine;
- R₅ is a triazole group, and
- R₆ is D-glucopyranosyl.

7. A compound of formula (I) for use according to any one of claims 1 to 5, in which:
- X₁ and X₂ are -C(O)-NH-;
- A represents a C₁₂ saturated hydrocarbon chain;
- R₁ and R₂ are -(CH₂)ₙ-R₃-(CH₂)ₘ-R₄-(CH₂)ₚ-R₅-R₆;
- n = m = p = 1;
- R₃ is a triazole group;
- R₄ is thymidine;
- R₅ is a triazole group, and
- R₆ is D-glucopyranosyl.

8. A compound of formula (I) for use according to any one of claims 1 to 5, in which
- X₁ is -C(O)-NH- and X₂ does not exist,
- A represents a C₄-C₃₀ hydrocarbon chain, linear or branched, saturated or unsaturated, which is partially or completely halogenated,
- R₁ is -(CH₂)ₙ-R₃-(CH₂)ₘ-R₄-(CH₂)ₚ-R₅-R₆,
wherein R₃, R₄-, R₅, R₆, n, m and p are as defined in any one of claims 1 to 5.

9. A compound of formula (I) for use according to any one of claims 1 to 8, in which R₃ is covalently linked to R₄ to the carbon atom at position 5' of the ribose or deoxyribose ring of the nucleosidyl group.

10. A compound of formula (I) for use according any one of claims 1 to 5, which is selected from the group consisting of
- 1,12-bis-dodecanyl-5'-[(4-methylurea)-1*H*-1,2,3-triazol-1-yl]-N-3-[1-((β-D-glucopyranoside)-1H-1,2,3-triazole-4-yl)methyl]-5'-deoxy thymidine,
- 1,14-bis-tetradecanyl-5'-[(4-methylamide)-1*H*-1,2,3-triazol-1-yl]-N-3-[1-((β-D-glucopyranoside)-1H-1,2,3-triazole-4-yl)methyl]-5'-deoxy thymidine, and
- 5'-(2H,2H,3H,3H-perfluoroundecanamide-(methylene-triazolyl), 5-(triazolyl)-thymidine.

11. A compound of formula (I) for use according to any one of claims 1 to 10, which is in the form of a hydrogel.

12. A compound of formula (II)
R₄-(CH₂)ₚ-R₅-R₆ (II)
in which:
- p is 0 to 10;
- R₄ represents a nucleosidyl group;
- R₅ represents a heteroaryl group comprising 1 to 4 heteroatom(s);
- R₆ represents a residue of a cyclic carbohydrate or a derivative of the said carbohydrate,
for use in promoting angiogenesis.

13. A compound of formula (II) for use according to claim 12, for tissue regeneration.

14. A compound of formula (II) for use according to claim 12 or 13, for tissue regeneration assisted by a biomaterial.

15. A compound of formula (II) for use according to any one of claims 12 to 14, in which
- p = 1;
- R₄ is thymidine;
- R₅ is a triazole group, and
- R₆ is D-glucopyranosyl.
